(19) Europäisches Patentamt
European Patent Office
Office européen des brevets

(11) Publication number: **0 147 062**
**A2**

# (12) EUROPEAN PATENT APPLICATION

(21) Application number: 84308163.9

(22) Date of filing: 26.11.84

(51) Int. Cl.$^4$: C 07 H 17/08

(30) Priority: 02.12.83 JP 228219/83

(43) Date of publication of application: 03.07.85 Bulletin 85/27

(84) Designated Contracting States: AT BE CH DE FR GB IT LI LU NL SE

(71) Applicant: TAISHO PHARMACEUTICAL CO. LTD
24-1 Takata 3-chome Toshima-ku
Tokyo 171(JP)

(72) Inventor: Morimoto, Shigeo
121-9, Homura Yoshikawacho
Kitakatsushika-gun Saitama-ken(JP)

(72) Inventor: Adachi, Takashi
1-1-5-205, Aoba
Kuki-shi(JP)

(72) Inventor: Takahashi, Yoko
Gurin-haitsu 101 1216-10, Kawarabuki
Ageo-shi(JP)

(72) Inventor: Watanabe, Yoshiaki
Ogawadanchi 1-101 2786, Ogawahigashicho
Kodaira-shi(JP)

(72) Inventor: Sota, Kaoru
1158-11, Shimotomi
Tokorozawa-shi(JP)

(72) Inventor: Kikugawa, Yasuo
27-10, Nishisakado-3-chome
Kasado-shi(JP)

(74) Representative: Harrison, David Christopher et al,
MEWBURN ELLIS & CO 2/3 Cursitor Street
London EC4A 1BQ(GB)

**(54) Method for preparing 6-0-methyl-2'-0,N-Bis (Benzyloxycarbonyl)-n-Demethylerythromycin a.**

(57) A method for preparing 6-O-methyl-2'-O,N-bis (benzyloxycarbonyl)-N-demethylerythromycin A comprises methylating 2'-O,N-bis(benzyloxycarbonyl)-N-demethylerythromycin A with a methylating agent in the presence of potassium hydroxide or sodium hydroxide in a polar aprotic solvent.



Croydon Printing Company Ltd.

# METHOD FOR PREPARING 6-0-METHYL-2'-0,N-BIS(BENZYLOXYCARBONYL)-N-DEMETHYLERYTHROMYCIN A

## BACKGROUND OF THE INVENTION

### 1. FIELD OF THE INVENTION

The present invention relates to a method for preparing an intermediate of 6-O-methylerythromycin A. More particularly, it relates to a method for preparing 6-O-methyl-2'-O,N-bis(benzyloxycarbonyl)-N-demethylerythromycin A of the formula

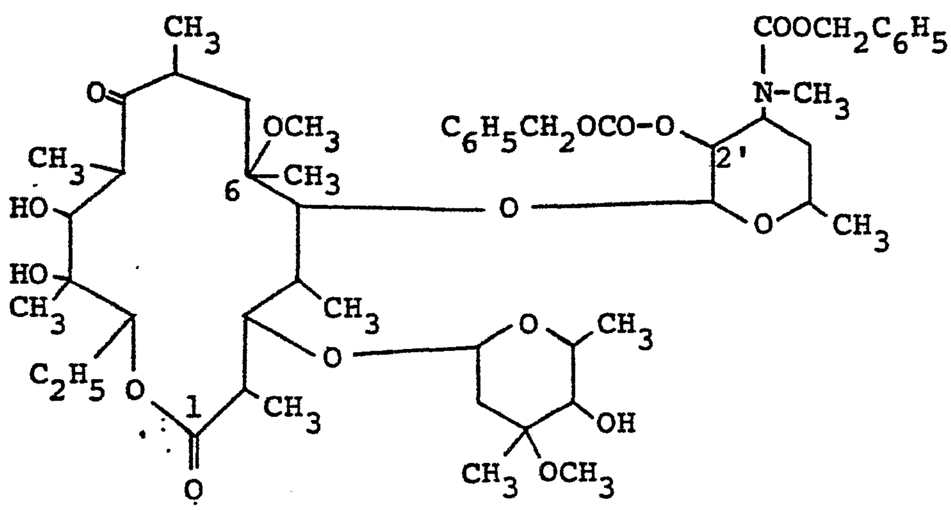

### 2. DESCRIPTION OF PRIOR ART

6-O-Methylerythromycin A has much higher <u>in vivo</u> antibacterial activity than erythromycin A and can be obtained by methylating a hydroxy group at the 6-position of erythronolide moiety of erythromycin A.

Erythromycin A, however, have five hydroxy groups in its molecule, and it is difficult to effect methylation itself of the particular hydroxy group of the compound. Especially, it is difficult to methylate a hydroxy group at the 6-position because this hydroxy group is tertiary.

Furthermore, erythromycin A is changeable because it is unstable under both the acidic and basic conditions, therefore, methylation of the hydroxy group should be carried out under an approximately neutral condition. For many restrictions described above, 6-O-methylerythromycin A was prepared with difficulty.

A method for preparing 6-O-methylerythromycin A has been reported in U.S. Patent No. 4,331,803. In this method, there are used an alkali metal hydride (e.g., lithium hydride, potassium hydride, sodium hydride and the like), an alkali metal amide (e.g., lithium amide, sodium amide and the like), butyl lithium, lithium diisopropylamide and the like. Therefore, this method requires anhydrous conditions during the reaction and troublesome reaction procedure, and are accompanied by proceeding the side-reactions. Furthermore, one of the problems in this method is the reproducibility of the yield and purity of the objective compound. In view of the above, this method is not proper as a method of mass production.

## SUMMARY OF THE INVENTION

As a result of earnest studies, the present inventors succeeded in methylating easily a tertiary hydroxy group at the 6-position of erythromycin A derivatives by using potassium hydroxide or sodium hydroxide, and found that this method provides the solution to the drawbacks of the prior art described above, and thus, the present invention has been completed.

According to the present invention, there is provided a method for preparing 6-O-methyl-2'-O,N-bis (benzyloxycarbonyl)-N-demethylerythromycin A (hereinafter referred to as Compound II) which comprises methylating 2'-O,N-bis(benzyloxycarbonyl)-N-demethylerythromycin A [hereinafter referred to as Compound I, which can be synthesized according to the method of E.H.Flynn et.al. in Journal of the American Chemical Society, 77, page 3104 (1955)] with a methylating agent in the presence of potassium hydroxide or sodium hydroxide in a polar aprotic solvent.

DETAILED DESCRIPTION OF PREFERRED EMBODIMENTS OF THE INVENTION

Potassium hydroxide and sodium hydroxide used in the present invention are those in the forms of powder or pellets which are commercially available, or a 9-12N aqueous solution. 0.9 to 1.3 molar equivalents of such base is added in one or several portions relative to the compound I. At the time when the base is added, it is required to add previously the methylating agent to the reaction solution.

On the other hand, the polar aprotic solvent includes dimethyl sulfoxide alone or a mixture of dimethyl sulfoxide with other organic solvents.

The organic solvents possible to mix with dimethyl sulfoxide are those which are inert to the reaction reagent, for example, dimethoxymethane, 1,2-dimethoxyethane, tetrahydrofuran, dioxane, ethyl acetate, butyl acetate,

acetone, methyl ethyl ketone, acetonitrile, dichloromethane, 1,2-dichloroethane, N,N-dimethylformamide, tetramethylene sulfoxide, hexamethylphosphoric triamide or a mixture thereof. Most preferred reaction solvents are dimethyl sulfoxide alone or a mixture of dimethyl sulfoxide and 1,2-dimethylethane with a ratio of from 1 : 1 to 1 : 2.

The methylating agent includes methyl halides such as methyl iodide, methyl bromide, methyl chloride and the like; dimethyl sulfate, methyl p-toluenesulfonate, methyl methanesulfonate and the like. Most preferred methylating agent is methyl iodide.

Although 1 - 8 molar equivalents of the methylating agent can be used relative to the compound I, it is usually sufficient to use 1 - 3 molar equivalents of it. It is necessary to add the methylating agent to the reaction solution prior to the addition of the base.

The method of the present invention can be carried out as follows.

In the above-mentioned polar aprotic solvent, are dissolved the compound I and the methylating agent, the base is added under ice-cooling with stirring, and the mixture is stirred at 0°C to room temperature for 2 to 5 hours to proceed the reaction. The progress of the reaction can be traced by means of thin layer chromatography using silica gel or high speed liquid chromatography.

When dimethyl sulfoxide is used as a polar aprotic solvent, it is preferred to use 1 to 1.2 moles of methyl iodide and 1 to 1.1 moles of potassium hydroxide per mole

of the compound I. When a mixture of dimethyl sulfoxide and 1,2-dimethoxyethane (1 : 1) is used, it is desired to use 1.0 to 4.0 moles of methyl iodide and 0.9 to 1.2 moles of potassium hydroxide per mole of the compound I.

After completion of methylation,

(1) the reaction solution is poured into water whose amount is 5 to 10 times that of the solution, and the formed precipitate is collected by filtration, or

(2) to the reaction solution, is added an amine (e.g., trimethylamine, triethylamine, dimethylamine, morpholine, ammonia and the like) in order to change an excess of the methylating agent to its quaternary salt. The mixture is poured into water, and extracted with ethyl acetate and the like to give the crude of the compound II.

Subsequently, the crude compound II is dissolved in dichloromethane, the solution is applied to silica gel column chromatography (dichloromethane as an eluting solvent), and the fractions containing the compound II are collected. The compound II in the considerably pure form can be obtained from these fractions. Although the compound II thus obtained is of 80 to 90% purity, generally it can be used as such for preparation of 6-O-methylerythromycin A as an intermediate without a further purification.

If necessary, the compound II thus obtained is purified by silica gel column chromatography [a mixture of chloroform and methanol (100 : 1) as an eluting solvent] to give the compound II as a white foam, which is then recrystallized from an aqueous ethanol (the ratio of ethanol

to water is 2 : 1) to give the compound II as needles.

The compound II thus obtained can be used advantageously for the preparation of 6-O-methylerythromycin A.

Namely, the compound II is dissolved in ethanol, an acetic acid-sodium acetate buffer solution (2.5 M, pH 5) is added, and the mixture is stirred in the presence of palladium black or palladium on carbon at ambient temperature under atmospheric pressure in a hydrogen atmosphere so that catalytic reduction takes place to eliminate benzyloxycarbonyl groups at the 2'-O- and N-positions of the compound II. Subsequently, an aqueous formaldehyde solution is added to the mixture so that a further catalytic reduction takes place to effect N-methylation of the compound II.

After completion of the reduction, the catalyst is separated by filtration, the filtrate is poured into water whose amount is 3 to 10 times that of the filtrate, and the mixture is adjusted to pH 10 to 10.3 with an aqueous alkaline solution. The resulting precipitate is collected by filtration, washed with water and recrystallized from ethanol to give 6-O-methylerythromycin A.

As stated above, according to the method of the present invention, methylation of the hydroxy group can be carried out under mild reaction conditions without the need of anhydrous conditions. Also, it can be carried out in a high safety because of no evolution of any dangerous combustible gases during the proceeding of the reaction.

Furthermore, according to the present invention, it is easy to operate the reaction procedure, the side-

reactions hardly takes place, and it is very easy to achieve the purification.

Accordingly, the method of the present invention is very useful as an industrial method for preparing an intermediate of 6-O-methylerythromycin A.

The present invention will be more concretely illustrated by the following Examples which show the preparations of the compound II and the following Referential Examples which show the preparations of 6-O-methylerythromycin A by using the compound II.

Example 1

In 1000 mℓ of a mixture of dimethyl sulfoxide and 1,2-dimethoxyethane (1 : 1), were dissolved 98.8 g (0.1 mole) of the compound I and 15 mℓ of methyl iodide, and 7.26 g (1.1 molar equivalents) of 85% potassium hydroxide powder was added in one portion under ice-cooling with stirring, and then the mixture was stirred at room temperature for 2 hours.

The reaction solution was poured into 5000 mℓ of water with stirring, and the resulting precipitate was collected by filtration, washed with water and dried to give 102 g of a crude product.

The crude product was dissolved in 100 mℓ of dichloromethane, the solution was subjected to a silica gel column chromatography (500 g of silica gel 60 for column chromatography produced by E. Merck Darmstadt, 70 - 230 mesh; dichloromethane as an eluting solvent). Thin layer

chromatography analysis (precoated thin layer chromatography plate of silica gel 60 $F_{254}$ produced by E. Merck Darmstadt, 0.25mm in thickness; a mixture of ethyl acetate and dichloromethane (1 : 2) as a developing solvent; normal chamber without chamber saturation) was conducted, and the fractions showing Rf value 0.42 (Rf value of the starting material is 0.17) were collected and concentrated to dryness under reduced pressure to give 40.1 g of the compound II as a white foam.

A solution of 1 g of this product in 2 mℓ of chloroform was subjected to a silica gel column chromatography [200 g of silica gel 60 for column chromatography produced by E. Merck Darmstadt, 70 - 230 mesh, column size 30 mmϕ x 570 mm, a mixture of chloroform and methanol (100 : 1) as an eluting solvent]. The eluates were analyzed by means of thin layer chromatography [precoated thin layer chromatography plate of silica gel 60 $F_{254}$ produced by E. Merck Darmstadt, 0.25 mm in thickness; a mixture of chloroform and methanol (30 : 1) as a developing solvent; normal chamber without chamber saturation], and the fractions showing Rf value 0.32 were collected. The solvent was evaporated under reduced pressure to give 0.85 g of a white foam like product.

Recrystallization of the product from a mixture of ethanol and water (2 : 1) gave 0.78 g of the compound II as needles.

m.p. 105 - 110°C

Elementary analysis for $C_{53}H_{79}NO_{17}$ (Molecular weight = 1002.17)

Calcd. (%): C, 63.52, H, 7.95, N, 1.40

Found (%): C, 63.24, H, 7.55, N, 1.41

$IR\nu_{max}^{KBr}$ $cm^{-1}$: 3420, 1745 (sh), 1735 (sh), 1730, 1700 (sh), 1685

$^{1}$H-NMR ($CDCl_3$) $\delta$ = 3.00 (3H, s, 6-OC$\underline{H}_3$)

$^{13}$C-NMR ($CDCl_3$) $\delta$ = 50.43 (C-6-O$\underline{C}H_3$)

Example 2

In 800 mℓ of a mixture of dimethyl sulfoxide and 1,2-dimethoxyethane (1 : 1), were dissolved 120 g (0.122 mole) of the compound I and 20 mℓ of methyl iodide, and 3.76 g (0.47 molar equivalent) of 85% potassium hydroxide powder was added under ice-cooling with stirring. After stirring the mixture for 30 minutes, a further 3.76 g (0.47 molar equivalent) of 85% potassium hydroxide powder was added, and the mixture was stirred for 2 hours.

To the reaction solution, was added dropwise 40 mℓ of triethylamine at 0 - 10°C, and the mixture was stirred for 30 minutes and then poured into a mixture of 3000 mℓ of ethyl acetate and 1000 mℓ of a saturated aqueous sodium chloride solution. After stirring the mixture, the ethyl acetate layer was separated.

The ethyl acetate layer was washed twice with 1000 mℓ of a saturated aqueous sodium chloride solution, dried over anhydrous sodium sulfate, and concentrated to dryness under reduced pressure to give 118 g of a foam like product.

Subsequently, the product was treated by the similar procedure to that of Example 1 to give 47.4 g of the compound II.

Example 3

In 60 mℓ of dimethyl sulfoxide, were dissolved 1.98 g (0.002 mole) of the compound I and 0.15 mℓ of methyl iodide, 146 mg (1.1 molar equivalents) of 85% potassium hydroxide powder was added, and the mixture was stirred at room temperature for 5 hours.

Subsequently, the mixture was treated by the similar procedure to that of Example 1 to give 0.88 g of the compound II.

Example 4

In 600 mℓ of a mixture of dimethyl sulfoxide and 1,2-dimethoxymethane (1 : 1), were dissolved 60 g (0.06 mole) of the compound I and 15 mℓ of methyl iodide, 6.1 mℓ (1.2 molar equivalents) of a 12N aqueous potassium hydroxide solution was added, and the mixture was stirred at room temperature for 3 hours.

Subsequently, the mixture was treated by the similar procedure to that of Example 1 to give 23 g of the compound II.

Example 5

In 60 mℓ of a mixture of dimethyl sulfoxide and 1,2-dimethoxyethane (1 : 1), were dissolved 6 g (0.006 mole)

of the compound I and 0.75 mℓ of methyl iodide, 281 mg (1.1 molar equivalents) of 95% sodium hydroxide powder was added, and the mixture was stirred at room temperature for 2 hours.

Subsequently, the mixture was treated by the similar procedure to that of Example 1 to give 2.4 g of the compound II.

Referential Example 1

In 300 mℓ of ethanol, was dissolved 20 g of the compound II, which was a part of the product obtained in Example 1. To this solution, were added 35 mℓ of aqueous solution containing 1.48 mℓ of glacial acetic acid and 5.06 g of sodium acetate and 1 g of palladium black, and the mixture was stirred vigorously for 7 hours at ambient temperature under atmospheric pressure in a hydrogen atmosphere.

After the disappearance of the compound II in the reaction solution was confirmed by means of a thin layer chloromatography analysis [precoated thin layer chromatograpy plate of silica gel 60 $F_{254}$ produced by E. Merck Darmstadt; a mixture of chloroform, methanol and conc. ammonium hydroxide (8 : 2 : 0.01) (v/v/v) as a developing solvent], 20 mℓ of a 35% aqueous formaldehyde solution was added to the reaction solution, and the mixture was stirred for a further 7 hours in a hydrogen atmosphere.

After completion of the reaction, the catalyst was filtered off, the filtrate was poured into 1500 mℓ of ice-water, and the mixture was adjusted to pH 10.3 with 2N sodium hydroxide. The mixture was allowed to stand

overnight, and subsequently the resulting precipitate was collected by filtration and washed thoroughly with water. Recrystallization of the precipitate from ethanol was repeated twice to give 10.3 g of 6-O-methylerythromycin A as colorless needles.

Referential Example 2

In 500 mℓ of a mixture of dimethyl sulfoxide and 1,2-dimethoxyethane (1 : 1), were dissolved 98.8 g (0.1 mole) of the compound I and 15 mℓ of methyl iodide, and 7.26 g (1.1 molar equivalents) of 85% potassium hydroxide powder was added at room temperature with stirring, and the mixture was stirred at room temperature for 3 hours.

The reaction solution was poured into 2500 mℓ of water with stirring, and the resulting precipitate was collected by filtration, washed with water and dried. The precipitate was dissolved in 250 mℓ of ethyl acetate, and the solution was passed through a short silica gel column (100 g of silica gel) and eluted by 300 mℓ of ethyl acetate. The eluates were combined and concentrated under reduced pressure to give 100 g of a white foam like product.

To a solution of the product in 1500 mℓ of ethanol, were added an aqueous solution consisting of 7.4 mℓ of glacial acetic acid, 25.3 g of sodium acetate and 175 mℓ of water and 15 g of 10% palladium on carbon, the mixture was stirred vigorously for 7 hours at ambient temperature under atmospheric pressure in a hydrogen atmosphere, and subsequently 100 mℓ of a 35% aqueous formaldehyde solution was

1 added. The mixture was stirred in a hydrogen atmosphere for further 7 hours. After completion of the reaction, the mixture was treated by the similar procedure to that of Referential Example 1 to give 16.4 g of 6-O-methylerythromycin A.

CLAIMS:-

1. A method for preparing 6-O-methyl-2'-O,N-bis (benzyloxycarbonyl)-N-demethylerythromycin A which comprises methylating 2'-O,N-bis(benzyloxycarbonyl)-N-demethylerythromycin A with a methylating agent in the presence of potassium hydroxide or sodium hydroxide in a polar aprotic solvent.

2. A method according to Claim 1, wherein the methylating agent is methyl iodide, methyl bromide, methyl chloride, dimethyl sulfate, methyl p-toluenesulfonate, or methyl methanesulfonate.

3. A method according to Claim 2, wherein the methylating agent is methyl iodide.

4. A method according to Claim 1, 2 or 3, wherein the polar aprotic solvent is dimethyl sulfoxide alone or a mixture of dimethyl sulfoxide with dimethoxymethane, 1,2-dimethoxyethane, tetrahydrofuran, dioxane, ethyl acetate, butyl acetate, acetone, methyl ethyl ketone, acetonitrile, dichloromethane, 1,2-dichloroethane, N,N-dimethylformamide, tetramethylene sulfoxide or hexamethylphosphoric triamide.

5. A method according to Claim 4, wherein the polar aprotic solvent is dimethyl sulfoxide or a mixture of dimethyl sulfoxide and 1,2-dimethoxyethane.

6. A method according to any preceding Claim, wherein the amount of methylating agent is 1 to 8 molar equivalents relative to 2'-O,N-bis(benzyloxycarbonyl)-N-demethylerythromycin A.

7. A method according to any preceding Claim, wherein the amount of potassium hydroxide or sodium hydroxide is 0.9 to 1.3

molar equivalents relative to 2'-O,N-bis(benzyloxycarbonyl)-N-demethylerythromycin A.

8. A method according to any preceding Claim, wherein the methylation is conducted at 0°C to room temperature for 2 to 5 hours.

9. A method according to any preceding Claim which includes the additional step of removing the said benzyloxycarbonyl groups from the 2'-0 and N-positions of the 6-0-methyl-2'-0,N-bis (benzyloxycarbonyl)-N-demethylerythromycin A, and methylating the resultant product at the N-position to give 6-0-methylerythromycin A.